# EUROPEAN PATENT SPECIFICATION

(11) **EP 3 354 256 B1**
(45) Date of publication and mention of the grant of the patent: **09.09.2020**
(21) Application number: 16860237.3
(22) Date of filing: 27.10.2016
(51) Int. Cl.: A61K 8/60, A61K 8/63, A61Q 19/02, A61Q 19/00, A61K 8/02

(54) **SOYASAPONIN-CONTAINING COMPOSITION FOR SKIN WHITENING**
SOJASAPONINHALTIGE ZUSAMMENSETZUNG ZUR HAUTAUFHELLUNG
COMPOSITION CONTENANT DE LA SAPONINE DE SOJA POUR LE BLANCHIMENT DE LA PEAU

(30) Priority: 30.10.2015 KR 20150152272
(43) Date of publication of application: 01.08.2018
(73) Proprietor: Amorepacific Corporation, Seoul 04386 (KR)
(72) Inventor: HWANG, Jeong Ah, Yongin-si Gyeonggi-do 17074 (KR); KANG, Young Gyu, Yongin-si Gyeonggi-do 17074 (KR); KO, Jae Young, Yongin-si Gyeonggi-do 17074 (KR); LEE, Hae Kwang, Yongin-si Gyeonggi-do 17074 (KR); PARK, Jun Seong, Yongin-si Gyeonggi-do 17074 (KR)
(74) Representative: Brevalex
(86) International application number: PCT/KR2016/012158
(87) International publication number: WO 2017/074054

(56) References cited:
- CN-A- 102 397 172
- JP-A- 2004 131 431
- KR-A- 20120 002 076
- KR-A- 20140 037 107
- KR-A- 20140 039 809
- KR-A- 20140 039 809
- US-A1- 2012 088 939
- US-A1- 2012 088 939
- US-B1- 6 471 972
- MASAKAZU SHIRAIWA ET AL: "Composition and Structure of "Group A Saponin" in Soybean Seed", AGRICULTURAL AND BIOLOGICAL CHEMISTRY, vol. 55, no. 2, 8 February 1991 (1991-02-08), pages 315-322, XP055554417, JP ISSN: 0002-1369, DOI: 10.1080/00021369.1991.10870574

## Description

### [Technical Field]

The present invention relates to a composition for skin whitening containing soyasaponin Aa as an active ingredient and more particularly, to a composition which is very safe to the skin with no side effects by using, as an active ingredient, a soyasaponin which can be extracted from a plant, exhibits a significantly excellent whitening effect by inhibiting skin pigmentation by inhibiting melanin production in the skin, reduces pigmentation symptoms such as melasma and freckles or improves skin dullness and enables an even and bright skin tone.

### [Background Art]

Human skin color is determined by several factors including the activity of melanocytes that produce melanin pigment, the distribution of blood vessels, the thickness of skin, and the presence or absence of pigments inside and outside the human body, such as carotenoids and bilirubin. In particular, black pigment called melanin produced by the action of several enzymes such as tyrosinase in melanocyte is the most important factor. Genetic factors, physiological factors associated with hormone secretion, stress, etc. and environmental factors such as ultraviolet irradiation affect the formation of melanin pigment. Melanin exists in the skin and has an important function of protecting the body from ultraviolet rays and the like. However, excessively produced melanin is known to accelerate pigmentation and skin aging and to induce skin cancer. In order to treat or ameliorate skin pigmentation abnormalities or excessive melanin pigmentation caused by exposure to UV or the like, ascorbic acid, kojic acid, arbutin, hudroquinone, glutathione or derivatives thereof, substances having inhibitory activity against tyrosinase have been added to cosmetics or medicines. However, their use is restricted because of insufficient skin whitening effect, skin safety issue, and stability issue within formulations when added to cosmetics.

### [Prior Art Document]

### [Patent Document]

1. Korean Patent Laid-open Publication No. 10-2012-0083943 (published on July 27, 2012)

### [DETAILED DESCRIPTION OF THE INVENTION]

### [Technical Problem]

Thus, the present inventors have conducted intensive studies to find out substances exhibiting excellent skin whitening effect, and found that soyasaponin present in soybean can provide excellent skin whitening effect, while having less skin irritation, thereby completing the present invention.

Therefore, an object of the present invention is to provide a composition exhibiting excellent skin whitening effect by using soyasaponin.

### [Technical Solution]

In order to achieve the above object, the present invention provides a skin-whitening preparation composition for external use or a pharmaceutical composition comprising soyasaponin Aa as an active ingredient.

The present invention also provides use of soyasaponin Aa as a skin whitening agent in the preparation of a skin preparation composition for external use (specifically, a cosmetic composition or a pharmaceutical composition).

### [ADVANTAGEOUS EFFECTS]

The soyasaponin used in the present invention is a natural compound present in plants and is used as medicinal herbs. Thus, it can be applied to the skin without side effects and can provide excellent skin whitening effect, reduce pigmentation symptoms such as melasma and freckles, or improve skin dullness, and enables an even and bright skin tone.

### [Brief Description of Drawings]

FIG. 1 shows the structure of group A soyasaponin.
FIG. 2 shows group B soyasaponin, group E soyasaponin, and group DDMP soyasaponin.
FIG. 3 shows the degree of intracellular melanin synthesis increased by α-MSH.
FIG. 4 shows the degree of extracellular melanin synthesis increased by α-MSH.
FIG. 5 shows the result of measuring the tyrosinase inhibitory activity of soyasaponin Aa using the method for evaluating the cell extract tyrosinase inhibitory activity.

### [DETAILED DESCRIPTION OF THE EMBODIMENTS]

The present invention relates to a composition for skin whitening, comprising soyasaponin Aa as an active ingredient.

Saponins are a glycoside compound found in plant species and have a structure in which at least one saccharide molecule is bound to aglycone such as steroid or triterpenoid, and it is known that about 500 kinds of plants contain saponin. Many kinds of saponins are present even in soybeans, and soyasaponin, which is a saponin of soybean, has a complex and diverse molecular structure based on the aglycone structure. Specifically, soyasaponin is largely classified into group A soyasaponin, group B soyasaponin, and group E soyasaponin according to soyasapogenol A represented by Chemical Formula 1 below, soyasapogenol B represented by Chemical Formula 2 below, and soyasapogenol E represented by Chemical Formula 3 below, respectively. The group A soyasaponin is a bisdesmoside saponin in which saccharide chains are bound at two positions of C-3 and C-22, whereas the group B soyasaponin and group E soyasaponin are monodesmoside saponins in which a saccharide chain is bound at one position of C-3. Further, the group B soyasaponin is divided into the one in which DDMP (2,3-dihydro-2,5-dihydroxy-6-methyl-4H-pyran-4-one) is bound at the C-22 position, and the one in which no DDMP is bound. Among them, the one in which DDMP is bound may be classified as a DDMP soyasaponin apart from the group B soyasaponin.

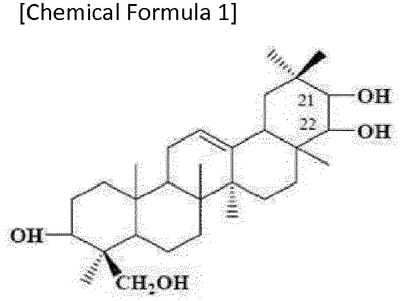

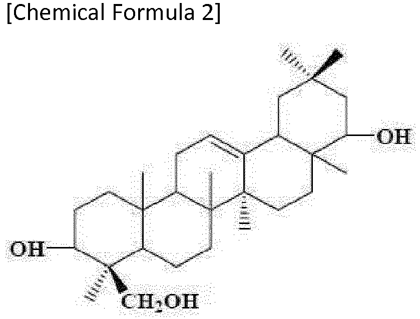

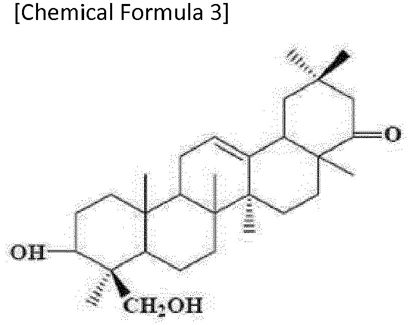

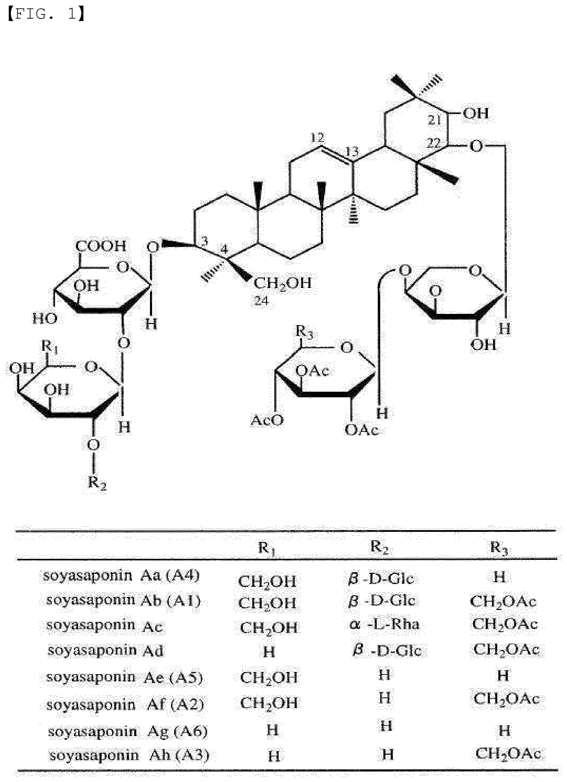

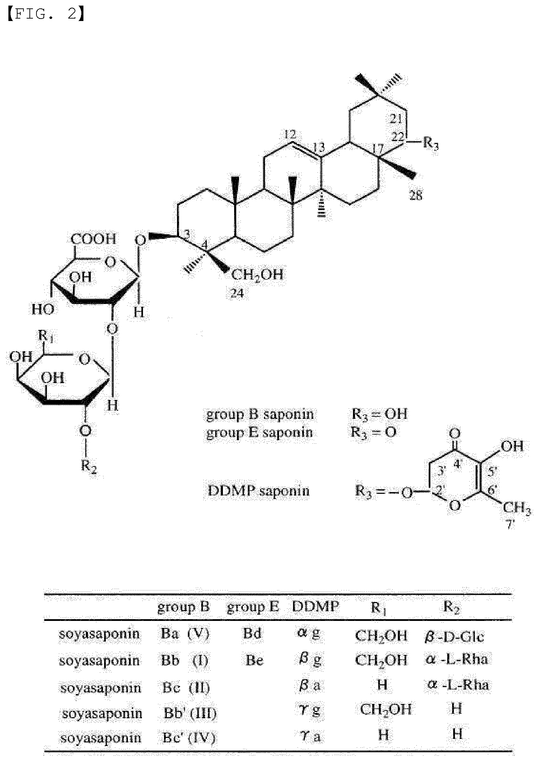

In addition, soyasaponin compounds corresponding to the group A soyasaponin may be classified as soyasaponins Aa, Ab, Ad, Ae, Af and the like depending on the scholar who named it, or may be classified as A1 to A6. Moreover, soyasaponin compounds corresponding to the group B soyasaponin in which no DDMP is bound may be classified as soyasaponins Ba, Bb, Bb', Bc and the like, or may be classified as soyasaponin I to V. Recently, as new soyasaponin compounds have been identified, the nomenclature has been expressed a little differently. FIG. 1 shows the structure of the group A soyasaponin, FIG. 2 shows the group B soyasaponin, group E soyasaponin, and DDMP soyasaponin. The soyasaponins shown in FIG. 1 and FIG. 2 can be summarized as shown in Table 1 below.

**[Table 1]**

| Group soyasaponin | Name of soyasaponin compound | Molecular structure of soyasaponin | Molecular weight |
|---|---|---|---|
| Group A | soyasaponin Aa(A4) | glc(1→2)gal(1→2)glcUA(1→3)-A-(22←1)ara(3←1)xyl(2,3,4-tri-O-acetyl) | 1364 |
| | soyasaponin Ab(A1) | glc(1→2)gal(1→2)glcUA(1→3)-A-(22←1)ara(3←1)glc(2,3,4,6-tetra-O-acetyl) | 1436 |
| | soyasaponin Ac | rha(1→2)gal(1→2)glcUA(1→3)-A-(22←1)ara(3←1)glc(2,3,4,6-tetra-O-acetyl) | 1420 |
| | soyasaponin Ad | glc(1→2)ara(1→2)glcUA(1→3)-A-(22←1)ara(3←1)glc(2,3,4,6-tetra-O-acetyl) | 1390 |
| | soyasaponin Ae(A5) | gal(1→2)glcUA(1→3)-A-(22←1)ara(3←1)xyl(2,3,4-tri-O-acetyl) | 1202 |
| | soyasaponin Af(A2) | gal(1→2)glcUA(1→3)-A-(22←1)ara(3←1)glc(2,3,4,6-tetra-O-acetyl) | 1274 |
| | soyasaponin Ag(A6) | ara(1→2)glcUA(1→3)-A-(22←1)ara(3←1)xyl(2,3,4-tri-O-acetyl) | 1172 |
| | soyasaponin Ah(A3) | ara(1→2)glcUA(1→3)-A-(22←1)ara(3←1)glc(2,3,4,6-tetra-O-acetyl) | 1244 |
| Group B | soyasaponin Ba(V) | glc(1→2)gal(1→2)glcUA(1→3)-B | 958 |
| | soyasaponin Bb (I) | rha(1→2)gal(1→2)glcUA(1→3)-B | 942 |
| | soyasaponin Bc(II) | rha(1→2)ara(1→2)glcUA(1→3)-B | 912 |
| | soyasaponin Bb'(III) | gal(1→2)glcUA(1→3)-B | 796 |
| | soyasaponin Bc'(IV) | ara(1→2)glcUA(1→3)-B | 766 |
| Group DDMP | soyasaponin αg | glc(1→2)gal(1→2)glcUA(1→3)-B-(22←2')-DDMP | 1084 |
| | soyasaponin βg | rha(1→2)gal(1→2)glcUA(1→3)-B-(22←2')-DDMP | 1068 |
| | soyasaponin βa | rha(1→2)ara(1→2)glcUA(1→3)-B-(22←2')-DDMP | 1038 |
| | soyasaponin γg | gal(1→2)glcUA(1→3)-B-(22←2')-DDMP | 922 |
| | soyasaponin γa | ara(1→2)glcUA(1→3)-B-(22←2')-DDMP | 892 |
| Group E | soyasaponin Bd | glc(1→2)gal(1→2)glcUA(1→3)-E | 956 |
| | soyasaponin Be | rha(1→2)gal(1→2)glcUA(1→3)-E | 940 |
| * "A" represents soyasapogenol A, "B" represents soyasapogenol B, "E" represents soyasapogenol E, "glc" represents β-D-glucopyranosyl, "gal" represents β-D-galactopyranosyl, "glcUA" represents β-D-glucuronopyranosyl, "ara" represents α-Larabinopyranosyl, "rha" represents α-L-rhamnopyranosyl, "xyl" represents β-D-xylopyranosyl, and "DDMP" represents 2,3-dihydro-2,5-dihydroxy-6-methyl-4H-pyran-4-one. | | | |

As used herein, the "derivative of soyasaponin" refers to a chemically modified soyasaponin, which means that a substituent has been modified using soyasaponin produced either by isolation from nature or by synthesis.

In the present invention, the soyasaponin can be prepared by isolation from natural materials or by a chemical synthesis method known in the art, and any soyasaponin compounds commercially available can be used.

The composition of the present invention contains, as an active ingredient, soyasaponin Aa.

The composition of the present invention may contain soyasaponin in an amount of 0.001% to 50% by weight, preferably 0.01% to 30% by weight, more preferably 0.1% to 10% by weight, based on the total weight of the composition. When the content is less than 0.001% by weight, the efficacy and effect due to the component are insufficient. If the content is more than 50% by weight, there is a problem in skin safety or shape.

As the composition according to the present invention contains soyasaponin, Aa it can inhibit melanin production and skin pigmentation, thereby providing an excellent skin whitening effect.

The composition according to the present invention is formulated as a skin preparation composition for external use.

The pharmaceutical composition according to the present invention may further contain a pharmaceutical adjuvant such as a preservative, a stabilizer, a hydrating agent or an emulsifying accelerator, a salt and/or a buffer for controlling osmotic pressure, etc., and other therapeutically useful substances, and may be prepared into various formulations for oral or parenteral administration in accordance with a conventional method.

The pharmaceutical composition according to the present invention can be administered via parenteral, rectal, topical, transdermal, subcutaneous routes or the like. The pharmaceutical composition according to the present invention can be, for example, administered topically to the skin.

Determination of the dose of the active ingredient is within the level of those skilled in the art, and the daily dose of a drug will vary depending on various factors, such as the progression of symptoms of a subject, time of onset, age, health condition, complications and the like. For adults, the composition can be typically administered at a dose of 1 *µ*g/kg to 200 mg/kg, preferably, 50 *µ*g/kg to 50 mg/kg by a split-dose method of once to thrice each day and the dose is not intended to limit the scope of the present invention in any way.

The composition according to the present invention may be a cosmetic composition. The external form of the cosmetic composition contains a cosmetically or dermatologically acceptable medium or base. It may be in any form suitable for topical application, for example, it may be provided in the form of solutions, gels, solids, paste anhydrous products, emulsions obtained by dispersing oil phase in aqueous phase, suspensions, microemulsions, microcapsules, microgranules, or ionic (liposomes) and non-ionic vesicle dispersants, or in the form of cream, skin toner, lotion, powder, ointment, spray or conceal stick. These compositions may be prepared according to a conventional method in the art. The composition according to the present invention can be used in the form of foam or as an aerosol composition further containing a compressed propellant.

When the formulation of the present invention is paste, cream, or gel, animal fiber, vegetable fiber, wax, paraffin, starch, tragacanth, cellulose derivatives, polyethylene glycol, silicon, bentonite, silica, talc, or zinc oxide, etc., can be used as the carrier ingredient.

When the formulation of the present invention is powder or spray, lactose, talc, silica, aluminum hydroxide, calcium silicate, or polyamide powder can be used as the carrier component, and particularly, in the case of spray, it may further include a propellant, such as chlorofluorohydrocarbon, propane/butane or dimethyl ester.

When the formulation of the present invention is a solution or an emulsion, solvents, solvating agents, or emulsifying agents are used as the carrier ingredient, and examples thereof include water, ethanol, isopropanol, ethyl carbonate, ethyl acetate, benzyl alcohol, benzyl benzoate, propylene glycol, 1,3-butylglycol oil, glycerol aliphatic ester, polyethylene glycol, or sorbitan fatty acid ester.

When the formulation of the present invention is a suspension, liquid diluting agents, such as water, ethanol, or propylene glycol, suspending agents, such as ethoxylated isostearyl alcohol, polyoxyethylene sorbitol ester, and polyoxyethylene sorbitan ester, and microcrystalline cellulose, aluminum meta-hydroxide, bentonite, agar, or tragacanth, etc., can be used as the carrier ingredient.

The composition of the present invention may further contain, in addition to the above-described components, functional additives and components contained in a general skin whitening composition. The functional additives may include components selected from the group consisting of water-soluble vitamins, oil-soluble vitamins, high molecular weight peptides, high molecular weight saccharides, sphingolipids and seaweeds extracts.

The cosmetic composition of the present invention may further be mixed with components contained in general cosmetic compositions, in addition to the above functional additives, if necessary. The mixing components which can be added additionally may include oil and fat components, humectants, emollient agents, surfactants, organic or inorganic pigments, organic powder, UV absorbents, preservatives, bactericides, antioxidants, vegetable extracts, pH adjusting agents, alcohols, pigments, flavoring agents, blood circulation promoters, cooling agents, adiaphoretics, purified water, or the like.

Further, the present invention relates to a skin preparation for external use including the composition, and the skin preparation for external use is a generic term that may include any substances applied to the skin exterior, and cosmetics and medicines of various formulations may be included therein.

In addition, the composition of the present invention may contain a skin absorption promoting substance to increase the effects of increasing skin whitening effect.

### [DETAILED DESCRIPTION OF THE EMBODIMENTS]

Hereinafter, the constitution and effects of the present invention will be described in more detail by way of Experimental Examples and Preparation Examples. However, these Experimental Examples and Preparation Examples are given for illustrative purposes only, and the scope of the invention is not intended to be limited by these Examples

### [Reference Example 1] Preparation of soyasaponin

In order to test the efficacy of the composition of the present invention, soyasaponin Aa was purchased from Chengdu Biopurity Phytochemicals Ltd., and used in the test.

### [Test Example 1] Evaluation of the degree of melanin production inhibition

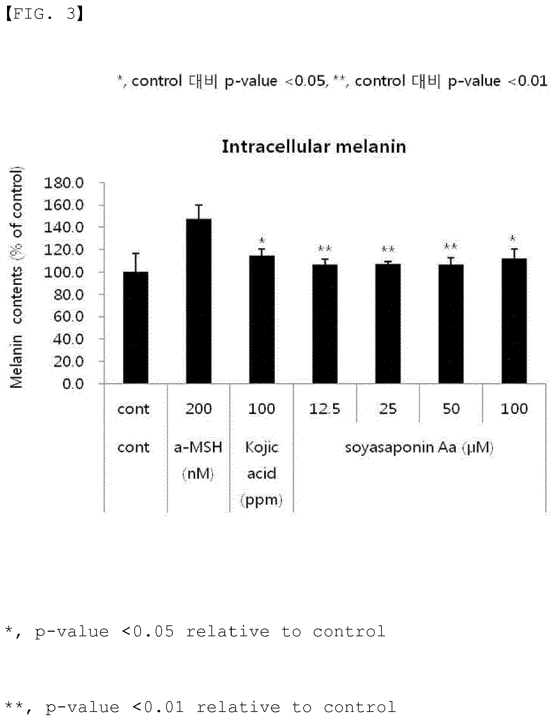

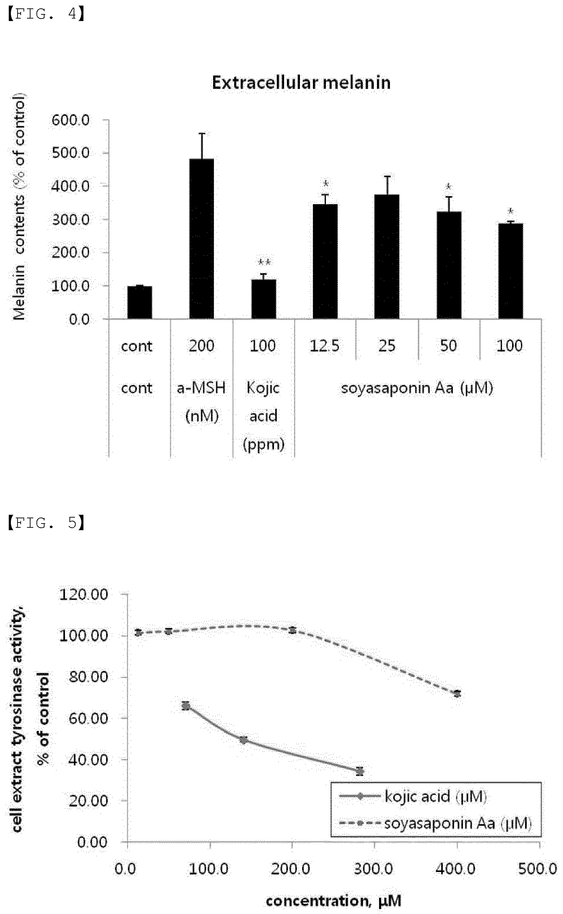

Murine melanoma (B16) cells purchased from the Korean Cell Line Bank were seeded into each well of a 12-well plate (5 × 10⁴ cells/well) in DMEM (Dulbecco's modified eagle's medium) containing FBS (fetal calf serum) or a medium having growth rates equivalent to or higher than those in the DMEM. The cells were cultured at 37°C with 5% CO₂ until they reached about 80% confluency. After cultivation, the medium was removed and samples were replaced in medium diluted with 12.5, 25, 50 or 100µM of soyasaponin Aa, or 100 ppm (corresponding to 700 µM) of kojic acid, and then cultured at 37°C with 5% CO₂ for 3 days. At this time, α-MSH (melanin stimulation hormone), which is a substance that allows B16 cells to better form melanin during cultivation, was included together in the medium of the other treatment groups except for the untreated control group. In addition, in order to compare the efficacies during the treatment of soyasaponin Aa or kojic acid, the B16 cells were cultured even in a medium containing only α-MSH without containing soyasaponin Aa or kojic acid. The concentration range of the samples was determined through toxicity tests. Cells removing medium were washed with PBS (phosphate buffer saline), and 100 µl of 1 M NaOH containing 10% DMSO or cell lysis buffer was added thereto to obtain intracellular melanin in a thermostat at 60°C. The obtained solution was measured for the absorbance at 490 nm using an ELISA reader (Synergy 2, BioTek. USA), and the amount of protein was determined by the Bradford assay, and then the amount of melanin per cell constant number or the amount of melanin per protein constant number was estimated. Melanin production activities (%) of soyasaponin Aa and kojic acid at various concentrations as compared to the control not treated with the sample are shown in FIG. 3 (the degree of extracellular melanin synthesis) and FIG. 4 (the degree of extracellular melanin synthesis).

As shown from FIG. 3 and FIG. 4, it was confirmed that soyasaponin Aa effectively inhibited the synthesis of melanin.

### [Test Example 1] Evaluation of inhibitory effect on tyrosinase activity

Tyrosinase is an important enzyme that regulates the first step that is performed to produce melanin in the process of pigmentation, and tyrosinase inhibitory activity is known to be an important efficacy mechanism for whitening. Therefore, in this test example, the ability of soyasaponin to inhibit the activity of tyrosinase was evaluated using a method for inhibiting the activity of cell extract tyrosinase. The method for evaluating the cell extract tyrosinase inhibitory activity is based on a phenomenon that melanocyte-derived tyrosinase causes a color development reaction by allowing substrate DOPA (3,4-dihydroxyphenylalanine; colorless) to form dopacrome (reddish brown).

Specific experimental methods are as follows. Kojic acid as a positive control and soyasaponin Aa as an evaluation material were prepared by concentrating four times the concentration to be evaluated. Then, the reaction mixture (pH 6.8, 0.1 M potassium phosphate buffer, melan-a cell extract mixture) was prepared in a 96 well plate. Since there may be colors depending on the evaluation material, a reaction mixture containing no cell extract liquid was made into a vehicle for background removal. The plate containing the reaction mixture was covered using a lid or a sheet and incubated at 37°C for 1 hour. Then, substrate DOPA (2 mg/ml) was added to each well in an amount of 100 µl, and the plate was covered with a lid or a sheet, followed by reaction at 37°C for 10 minutes. Absorbance was measured at 475 nm using an ELISA reader (Synergy2, BioTek, USA) at intervals of 10 minutes until sufficient color development was occurred. The results are shown in FIG. 5.

As shown in FIG. 5, the activity of tyrosinase was effectively inhibited during the treatment of soyasaponin Aa.

### [Formulation Example 1] Skin Lotion

Skin lotion was prepared according to a conventional method with the composition shown in Table 2 below.

**[Table 2]**

| | Content (wt%) |
|---|---|
| Soyasaponin Aa | 2.0 |
| Glycerin | 3.0 |
| Butylene glycol | 2.0 |
| Propylene glycol | 2.0 |
| Carboxyvinyl polymer | 0.1 |
| PEG 12 nonylphenyl ether | 0.2 |
| Polysolvate 80 | 0.4 |
| Ethanol | 10.0 |
| Triethanol amine | 0.1 |
| Preservative, pigment, flavoring agent | Adequate |
| Purified water | Residual |

### [Formulation Example 2] Nutritive cream

Nutritive cream was prepared according to a conventional method with the composition shown in Table 3 below.

**[Table 3]**

| | Content (wt%) |
|---|---|
| Soyasaponin Aa | 2.0 |
| Polysorvate 60 | 1.5 |
| Sorbitan sesquioleate | 0.5 |
| PEG 60 hardened castor oil | 2.0 |
| Liquid paraffin | 10.0 |
| Squalane | 5.0 |
| Caprylic/capric triglyceride | 5.0 |
| Glycerin | 5.0 |
| Butylene glycol | 3.0 |
| Propylene glycol | 3.0 |
| Triethanol amine | 0.2 |
| Preservative, pigment, flavoring agent | Adequate |
| Purified water | Residual |

### [Formulation Example 3] Massage cream

Massage cream was prepared according to a conventional method with the composition shown in Table 4 below.

**[Table 4]**

| | Content (wt%) |
|---|---|
| Soyasaponin Aa | 1.0 |
| Bees wax | 10.0 |
| Polysorvate 60 | 1.5 |
| PEG 60 hardened castor oil | 2.0 |
| Sorbitan sesquioleate | 0.8 |
| Liquid paraffin | 40.0 |
| Squalane | 5.0 |
| Caprylic/capric triglyceride | 4.0 |
| Glycerin | 5.0 |
| Butylene glycol | 3.0 |
| Propylene glycol | 3.0 |
| Triethanol amine | 0.2 |
| Preservative, pigment, flavoring agent | Adequate |
| Purified water | Residual |

### [Formulation Example 4] Pack

Pack was prepared according to a conventional method with the composition shown in Table 5 below.

**[Table 5]**

| | Content (wt%) |
|---|---|
| Soyasaponin Aa | 10.0 |
| Polyvinyl alcohol | 13.0 |
| Sodium carboxymethyl cellulose | 0.2 |
| Glycerin | 5.0 |
| Allantoin | 0.1 |
| Ethanol | 6.0 |
| PEG 12 nonylphenyl ether | 0.3 |
| Polysorvate 60 | 0.3 |
| Preservative, pigment, flavoring agent | Adequate |
| Purified water | Residual |

### [Formulation Example 5] Gel

Gel was prepared according to a conventional method with the composition shown in Table 6 below.

**[Table 6]**

| | Content (wt%) |
|---|---|
| Soyasaponin Aa | 0.5 |
| Ethylene diamine sodium acetate | 0.05 |
| Glycerin | 5.0 |
| Carboxy vinyl polymer | 0.3 |
| Ethanol | 5.0 |
| PEG 60 hardened castor oil | 0.5 |
| Triethanol amine | 0.3 |
| Preservative, pigment, flavoring agent | Adequate |
| Purified water | Residual |

### [Formulation Example 6] Ointment

Ointment was prepared according to a conventional method with the composition shown in Table 7 below.

**[Table 7]**

| | Content (wt%) |
|---|---|
| Soyasaponin Aa | 0.1 |
| Glycerin | 8.0 |
| Butylene glycol | 4.0 |
| Liquid paraffin | 15.0 |
| Beta glucan | 7.0 |
| Carbomer | 0.1 |
| Caprylic/capric triglyceride | 3.0 |
| Squalane | 1.0 |
| Cetearyl glucoside | 1.5 |
| Sorbitan stearate | 0.4 |
| Cetearyl glucoside | 1.0 |
| Bees wax | 4.0 |
| Preservative, pigment, flavoring agent | Adequate |
| Purified water | Residual |

Although specific parts of the present invention have been described in detail, it will be apparent to those skilled in the art that these specific techniques are merely a preferred embodiment and that the scope of the present invention is not limited thereto. Therefore, the substantial scope of the present invention will be defined by the accompanying claims and their equivalents.

## Claims

1. External use composition comprising soyasaponin Aa as an active ingredient for use in a method for treating or ameliorating skin pigmentation abnormalities or excessive melanin pigmentation.

2. External use composition for the use of claim 1, wherein the soyasaponin Aa is contained in an amount of 0.001% to 50% by weight based on the total weight of the composition.

3. External use composition for the use of claim 1, wherein the soyasaponin Aa is contained in an amount of 0.1% to 10% by weight based on the total weight of the composition.

4. External use composition for the use of claim 1, wherein the composition is used for improving complexion and skin tone.

5. Non-therapeutic (cosmetic) use of an external non-therapeutic (cosmetic) composition comprising a soyasaponin Aa as an active ingredient for skin whitening.

6. Non-therapeutic (cosmetic) use of claim 5, wherein the soyasaponin Aa is contained in an amount of 0.001% to 50% by weight based on the total weight of the composition.

7. Non-therapeutic (cosmetic) use of claim 5, wherein the soyasaponin Aa is contained in an amount of 0.1% to 10% by weight based on the total weight of the composition.

8. Non-therapeutic (cosmetic) use of claim 5, wherein the composition is used for improving complexion and skin tone.

9. Non-therapeutic (cosmetic) use of claim 5, wherein the composition is formulated in the form of cream, skin, lotion, powder, ointment, spray or conceal stick.

## Patentansprüche

1. Zusammensetzung zur externen Verwendung, die Sojasaponin Aa als einen aktiven Inhaltsstoff zur Verwendung in einem Verfahren zur Behandlung oder Verbesserung von Abnormalitäten der Hautpigmentierung oder einer exzessiven Melaninpigmentierung umfasst.

2. Zusammensetzung zur externen Verwendung für die Verwendung nach Anspruch 1, wobei das Sojasaponin Aa in einer Menge von 0.001 Gewichts-% bis 50 Gewichts-% enthalten ist, basierend auf dem Gesamtgewicht der Zusammensetzung.

3. Zusammensetzung zur externen Verwendung für die Verwendung nach Anspruch 1, wobei das Sojasaponin Aa in einer Menge von 0.1 Gewichts-% bis 10 Gewichts-% enthalten ist, basierend auf dem Gesamtgewicht der Zusammensetzung.

4. Zusammensetzung zur externen Verwendung für die Verwendung nach Anspruch 1, wobei die Zusammensetzung zur Verbesserung der Komplexierung und des Hauttonus verwendet wird.

5. Nicht-therapeutische (kosmetische) Verwendung einer externen nichttherapeutischen (kosmetischen) Zusammensetzung, die Sojasaponin Aa als einen aktiven Inhaltsstoff zur Hautaufhellung umfasst.

6. Nicht-therapeutische (kosmetische) Verwendung nach Anspruch 5, wobei das Sojasaponin Aa in einer Menge von 0.001 Gewichts-% bis 50 Gewichts-% enthalten ist, basierend auf dem Gesamtgewicht der Zusammensetzung.

7. Nicht-therapeutische (kosmetische) Verwendung nach Anspruch 5, wobei das Sojasaponin Aa in einer Menge von 0.1 Gewichts-% bis 10 Gewichts-% enthalten ist, basierend auf dem Gesamtgewicht der Zusammensetzung.

8. Nicht-therapeutische (kosmetische) Verwendung nach Anspruch 5, wobei die Zusammensetzung zur Verbesserung der Komplexierung und des Hauttonus verwendet wird.

9. Nicht-therapeutische (kosmetische) Verwendung nach Anspruch 5, wobei die Zusammensetzung in der Form von Creme, Haut, Lotion, Puder, Salbe, Spray oder Deckstift formuliert ist.

## Revendications

1. Composition pour usage externe comprenant une saponine de soja Aa en tant que principe actif pour une utilisation dans un procédé de traitement ou d'amélioration des anomalies de pigmentation cutanée ou d'une pigmentation excessive par la mélanine.

2. Composition pour usage externe pour l'utilisation selon la revendication 1, dans laquelle la saponine de soja Aa est contenue en une quantité de 0,001 % à 50 % en poids sur la base du poids total de la composition.

3. Composition pour usage externe pour l'utilisation selon la revendication 1, dans laquelle la saponine de soja Aa est contenue en une quantité de 0,1 % à 10 % en poids sur la base du poids total de la composition.

4. Composition pour usage externe pour l'utilisation selon la revendication 1, dans laquelle la composition est utilisée pour améliorer le teint et le ton de la peau.

5. Utilisation non thérapeutique (cosmétique) d'une composition externe non thérapeutique (cosmétique) comprenant une saponine de soja Aa en tant que principe actif pour blanchir la peau.

6. Utilisation non thérapeutique (cosmétique) selon la revendication 5, dans laquelle la saponine de soja Aa est contenue en une quantité de 0,001 % à 50 % en poids sur la base du poids total de la composition.

7. Utilisation non thérapeutique (cosmétique) selon la revendication 5, dans laquelle la saponine de soja Aa est contenue en une quantité de 0,1% à 10 % en poids sur la base du poids total de la composition.

8. Utilisation non thérapeutique (cosmétique) selon la revendication 5, dans laquelle la composition est utilisée pour améliorer le teint et le ton de la peau.

9. Utilisation non thérapeutique (cosmétique) selon la revendication 5, dans laquelle la composition est formulée sous la forme d'une crème, d'une peau, d'une lotion, d'une poudre, d'une pommade, d'un spray ou d'un bâtonnet correcteur de teint.
